(19) Europäisches Patentamt
European Patent Office
Office européen des brevets

(11) Veröffentlichungsnummer: **0 130 141**
**B1**

(12) **EUROPÄISCHE PATENTSCHRIFT**

(45) Veröffentlichungstag der Patentschrift: **12.09.90**

(21) Anmeldenummer: **84730070.4**

(22) Anmeldetag: **21.06.84**

(51) Int. Cl.$^5$: **C 07 D 471/04,** A 61 K 31/435 // (C07D471/04, 221:00, 209:00)

(54) **Neue beta-Carboline, Verfahren zu ihrer Herstellung und ihre Verwendung als Arzneimittel (H).**

(30) Priorität: **23.06.83 DE 3322894**

(43) Veröffentlichungstag der Anmeldung: **02.01.85 Patentblatt 85/01**

(45) Bekanntmachung des Hinweises auf die Patenterteilung: **12.09.90 Patentblatt 90/37**

(84) Benannte Vertragsstaaten: **AT BE CH DE FR GB IT LI LU NL SE**

(56) Entgegenhaltungen:
**EP-A-0 030 254**
**EP-A-0 054 507**

**CHEMICAL ABSTRACTS, Band 99, 1983, Seite 742, Nr. 194831j, Columbus, Ohio, US; G. NEEF u.a.: "Synthesis of 4-substituted beta-carbolines"**

(73) Patentinhaber: **SCHERING AKTIENGESELLSCHAFT Berlin und Bergkamen Müllerstrasse 170/178 Postfach 65 03 11 D-1000 Berlin 65 (DE)**

(72) Erfinder: **Huth, Andreas, Dr.**
**Kyllmannstrasse 15**
**D-1000 Berlin 39 (DE)**
Erfinder: **Schmiechen, Ralph, Dr.**
**Bayernring 27**
**D-1000 Berlin 42 (DE)**
Erfinder: **Seidelmann, Dieter, Dr.**
**Stierstrasse 14**
**D-1000 Berlin 41 (DE)**
Erfinder: **Rahtz, Dieter, Dr.**
**Krottnauer Strasse 24a**
**D-1000 Berlin 28 (DE)**
Erfinder: **Engelstoft, Mogens, Dr.**
**Mosegaard Park 121**
**DK-3500 Vaerloese (DK)**
Erfinder: **Braerstrup, Claus Thyco, Dr.**
**Frederiksborgvej 78**
**DK-4000 Roskilde (DK)**

**EP 0 130 141 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue substituierte β-Carboline, Verfahren zu ihrer Herstellung und ihre Verwendung als Arzneimittel gemäß den Ansprüchen.

Die erfindungsgemäßen Verbindungen besitzen wertvolle pharmakologische Eigenschaften. Sie beeinflussen insbesondere das Zentralnervensystem und eignen sich somit als Psychopharmaka.

β-Carboline, die in 3-Stellung mit einem substituierten 5-Oxidiazolylrest, oder mit einem Alkyloxycarbonylrest substituiert sind, werden in EP—A—30254 oder EP—A—54507 beschrieben.

Die neuen β-Carboline haben die allgemeine Formel I

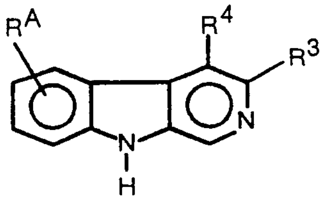

(I),

worin

$R^3$ einen Oxadiazolylrest der Formel

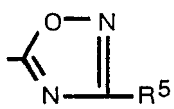

wobei $R^5$ niederes Alkyl mit bis zu 5 C-Atomen bedeutet, oder die Gruppierung —$COOR^6$, wobei $R^6$ niederes Alkyl, mit bis zu 7 C-Atomen bedeutet,

$R^4$ Wasserstoff, niederes Alkyl mit bis zu 3 C-Atomen oder $CH_2OR^9$, wobei $R^9$ niederes Alkyl mit bis zu 3 C-Atomen darstellt, und

$R^A$ die Gruppierung —$COOR^{10}$, wobei $R^{10}$ einen niederen Alkylrest mit bis zu 5 C-Atomen oder die Benzylgruppe darstellt

oder die Gruppierung

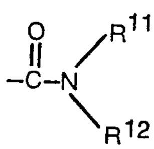

wobei $R^{11}$ und $R^{12}$ gleich oder verschieden sind und Wasserstoff oder niederes Alkyl oder Alkenyl darstellen oder gemeinsam einen stckstoffhaltigen 6-gliedrigen Ring darstellen, wobei $R^4$ nicht Wasserstoff oder niederes Alkyl bedeutet, falls $R^A$ und $R^3$ COO-nieder-Alkyl sind.

Unter Alkyl sind sowohl gerad- als auch verzweigtkettige Reste zu verstehen. Beispielsweise seien genannt Methyl, Ethyl, Propyl, iso-Propyl, n-Butyl, iso-Butyl und tert.-Butyl.

In 4-Stellung weisen die neuen β-Carboline entweder Wasserstoff, niederes Alkyl wie Methyl oder Ethyl oder eine Niederalkoxymethylgruppe auf.

Der Substituent $R^A$ kann sich in 5- oder 6-Stellung befinden, wobei die 6-Stellung bevorzugt ist.

Es ist bekannt, daß bestimmte Stellen im zentralen Nervensystem von Vertebraten eine hohe spezifische Affinität für die Bindung von 1.4- und 1.5-Benzodiazepinen aufweisen (Squires, R. F. und Braestrup, C., Nature (London)266(1977) 734). Die Stellen werden Benzodiazepin-Rezeptoren genannt.

Es wurde gefunden, daß die erfindungsgemäßen substituierten β-Carboline, obwohl sie sich in ihrer chemischen Struktur von den Benzodiazepinen stark unterscheiden, überraschenderweise eine starke Affinität und Spezifität für die Bindung an die Benzodiazepin-Rezeptoren zeigen, indem sie radioaktivmarkiertes Flunitrazepam von diesen Benzodiazepin-Rezeptoren verdrängen.

Die Verdrängungsaktivität der erfindungsgemäßen Verbindungen ist in der nachfolgenden Tabelle als $IC_{50}$- und $ED_{50}$-Wert angegeben. Der $IC_{50}$-Wert gibt die Konzentration an, die eine 50%ige Verdrängung der spezifischen Bindung von $^3H$-Flunitrazepam (1,0 nM, 0°C) in Proben mit einem Gesamtvolumen von 0,55 ml einer Suspension von Hirnmembran, z.B. von Ratten bewirkt.

Die Verdrängungsaktivität wird im in-vitro Test wie folgt bestimmt: 0,5 ml einer Suspension von unbehandeltem Ratten-Haupthirn in 25 mM $KH_2PO_4$, pH = 7,1 (5—10 mg Gewebe/Probe) wird für 40—60 Minuten bei 0°C zusammen mit $^3H$-Diazepam (spezifische Aktivität 14,4 Ci/mMol, 1,9 nM) oder $^3H$-Flunitrazepam (spezifische Aktivität 87 Ci/mMol, 1,0 nM) inkubiert. Nach Inkubation wird die Suspension durch eine Glasfritte filtriert, der Rückstand 2mal mit kalter Pufferlösung gewaschen und die Radioaktivität am Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erhaltenen Werte wird der $IC_{50}$-Wert berechnet.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50% des Kontrollwertes bewirkt.

Der in-vivo Test wird wie folgt ausgeführt:

Gruppen von Mäusen wird die Versuchssubstanz bei unterschiedlichen Dosen und normalerweise subcutan injiziert. Nach 15 Minuten wird den Mäusen das $^{3}H$-Flunitrazepam intravenös verabreicht. Nach weiteren 20 Minuten werden die Mäuse getötet, ihre Vorderhirnhäute entfernt und die Radioaktivität der Vorderhirnhäute durch Scintillationszählung gemessen. Der $ED_{50}$-Wert wird mit Hilfe der Dosis/Wirkungs-Kurven bestimmt.

## TABELLE

Verdrängungsaktivität von substituierten ß-Carbolin-derivaten der Formel I

| Substituent $R^3$ | $R^4$ | $6\text{-}R^A$ | $IC_{50}$ ng/ml (in vitro) | $ED_{50}$ mg/kg (in vivo) |
|---|---|---|---|---|
| $CO_2Me$ | H | H *) | 1,9 | 22 |
| $CO_2Et$ | $CH_3$ | $CON(Allyl)_2$ | 0,4 | 9,5 |
| $CO_2Et$ | H | CO-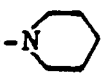 | 0,6 | 8,1 |
| $CO_2Et$ | $CH_2OCH_3$ | $CON(CH_3)_2$ | 1,3 | 12 |
| 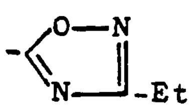 | H | CO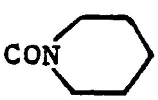 | 0,6 | 6,7 |

*) Nature *294* (1981) 472

Die erfindungsgemäßen Verbindungen erscheinen aufgrund ihrer biologischen Wirksamkeit als Psychopharmaka für die Humanmedizin geeignet. Sie können hier zu psychopharmazeutischen Präparationen, zum Beispiel für die orale und parenterale Anwendung, formuliert angewendet werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag, vorzugsweise 1—30 mg/Tag, angewendet.

Die Herstellung der erfindungsgemäßen Verbindungen der allgemeinen Formel I erfolgt nach an sich bekannten Methoden dadurch, daß man ein

β-Carbolinderivat der allgemeinen Formel II

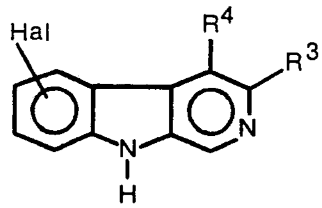

(II),

worin $R^3$ und $R^4$ die oben angegebene Bedeutung haben und Hal Brom oder Jod bedeutet, in Gegenwart einer organischen Base mit Kohlenmonoxid und einem organischen Alkohol der Formel $R^{10}OH$, wobei $R^{10}$, die obige Bedentung hat, oder einem stikstoffhaltigen 6-gliedrigem Ring unisetst

oder ein substituiertes β-Carbolinderivat der Formel III

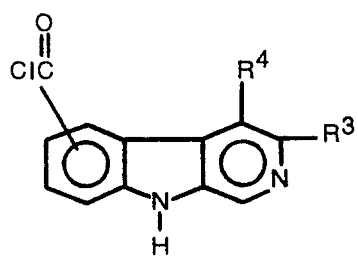

(III),

wobei $R^3$ und $R^4$ die oben angegebene Bedeutung haben, mit einem primären oder sekundären Amin der Formel $HNR^{11}R^{12}$, worin $R^{11}$ und $R^{12}$ die oben angegebene Bedeutung haben, umsetzt

oder eine substituierte β-Carbolin-3-carbonsäure der allgemeinen Formel IV

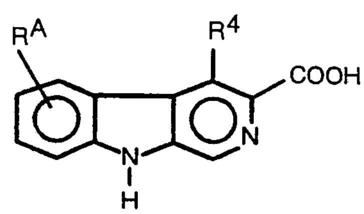

(IV),

worin $R^A$ und $R^4$ die oben angegebene Bedeutung haben, mit einem Amidoxim der allgemeinen Formel $R^5—C(=NOH)NH_2$, worin $R^5$ die oben angegebene Bedeutung hat, zum 5-Oxadiazolylderivat umsetzt und gegebenenfalls einen Ester eines aliphatischen Alkohols alkalisch hydrolysiert und gegebenenfalls die freie Säure mit einem Alkohol in gegenwart von Cäsiumcarbonat wieder verestert.

Zur Herstellung von Verbindungen der Formel I, in denen $R^A$ eine $R^{10}$-Oxycarbonylgruppe darstellt, wird das entsprechende 6-Halogen-β-carbolinderivat zusammen mit dem entsprechenden Alkohol der Formel $R^{10}OH$ in Gegenwart eines basischen Katalysators wie Tributylamin und einem Palladium(II)-salz

wie Palladium(II)-acetat unter Kohlenmonoxidatmosphäre bei Temperaturen oberhalb Raumtemperatur im Bereich von 100°C carboxyliert.

Zur Herstellung von Verbindungen der Formel I, in denen $R^A$ die Gruppierung $CONR^{11}R^{12}$ darstellt, wird das entsprechende β-Carbolin-6-carbonsäurechlorid in einem geeigneten inerten Lösungsmittel wie Tetrahydrofuran, Acetonitril, Methylenchlorid, Chloroform oder Dioxan zweckmäßigerweise unter Kühlung mit einem primären oder sekundären Amin der Formel $NHR^{11}R^{12}$ umgesetzt.

Zur Herstellung von Verbindungen der Formel I, in denen $R^3$ den 5-Oxadiazolylrest darstellt, wird die entsprechende freie β-Carbolin-3-carbonsäure mit einem Amidoxim der Formel $R^5—C(=NOH)NH_2$, wobei $R^5$ einen Niederalkylrest darstellt, in einem Lösungsmittel, das über 100°C siedet und gegenüber den Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie β-Carbolin-3-carbonsäure vor der Kondensationsreaktion in geeigneter Weise aktiviert. Hierzu kann die freie Säure in das gemischte Anhydrid, in den aktivierten Ester oder in das Chlorid überführt werden. Gut bewährt hat sich eine Aktivierung mit Imidazol/Thionylchlorid in einem aprotischen Lösungsmittel wie Dioxan, Tetrahydrofuran, Dimethylformamid oder N-Methylpyrrolidon bei Temperaturen zwischen 0 und 50°C, vorzugsweise Raumtemperatur.

Zur Herstellung einer freien β-Carbolin-6-carbonsäure kann eine Aralkylgruppe wie die Benzylgruppe wie bei dem β-Carbolin-6-carbonsäurebenzylgruppe wie bei dem β-Carbolin-6-carbonsäurebenzylester abhydriert werden. Hierzu wird der 6-Benzylester in methanolischer Salzsäure an Palladium hydriert. Bei dieser Methode wird eine gegebenenfalls vorhandene 3-Alkoxycarbonylgruppe am β-Carbolinmolekül nicht angegriffen.

Zur Herstellung einer freien μ-Carbolin-3-carbonsäure wird der entsprechende Ester in einem aliphastischen Alkohol wie Methanol oder Ethanol mit verdünntem wässrigem Alkali wie Natron- oder Kalilauge bei der Siedetemperatur des Reaktionsgemisches hydrolysiert.

Zur Veresterung einer freien β-Carbolin-3-carbonsäure wird diese mit Cäsiumcarbonat in das Cäsiumsalz überführt und anschließend mit dem entsprechenden Alkylhalogenid zur Reaktion gebracht.

Zur Umesterung wird der Entsprechende Ester der Formel I mit dem gewünschten Alkohol in Gegenwart katalytischer Mengen des entsprecheenden Natriumalkoholats oder Natriumhydrid 3—6 Stunden auf Temperaturen zwischen 60 und 120°C erhitzt. Gegebenenfalls kann die Umesterung auch mit diesem Alkohol in Gegenwart eines sauren Katalysators wie p-Toluolsulfonsäure, Salzsäure oder Kupfer-IIchlorid vorgenommen werden.

Herstellung des Ausgangsmaterials

A) *6-Jod-4-methyl-β-carbolin-3-carbonsäureethylester*

5,08 g 4-Methyl-β-carbolin-3-carbonsäureethylester werden in 40 ml Eisessig gelöst und mit 0,96 ml Wasser, 0,24 ml konzentrierter Schwefelsäure, 688 mg Jodsäure und 1,768 mg Jod versetzt. Die Mischung wird 3 Stunden auf 80°C erhitzt. Nach Abkühlen wird vom Ungelösten abgesaugt und das Filtrat eingedampft. Der Rückstand wird in Ethanol/Wasser aufgenommen. Die sich bildenden Kristalle werden abgesaugt, in 500 ml Essigester aufgenommen und mit 200 ml 1-n-Natronlauge 15 Minuten ausgerührt. Die organische Phase wird abgetrennt, eingedampft, der Rückstand in Essigester ausgerührt und abgesaugt. Man erhält 4,3 g 6-Jod-4-methyl-β-carbolin-3-carbonsäureethylester vom Schmelzpunkt 250—255°C.

In analoger Weise wird 4-Ethyl-6-jod-β-carbolin-3-carbonsäureethylester hergestellt.

B) *3-Ethoxycarbonyl-4-ethyl-β-carbolin-6-carbonsäurechlorid und Homologe*

590 mg 3-Ethoxycarbonyl-4-ethyl-β-carbolin-6-carbonsäure werden in 6 ml Thionylchlorid mit einem Tropfen Dimethylformamid 2,5 Stunden am Rückfluß gekocht Nach Eindampfen und Trocknen erhält man 664 mg 3-Ethoxycarbonyl-4-ethyl-β-carbolin-6-carbonsäurechlorid in Form des Hydrochlorids.

Des weiteren werden hergestellt:

3-Ethoxycarbonyl-4-methyl-β-carbolin-6-carbonsäurechlorid,

3-Ethoxycarbonyl-4-methoxymethyl-β-carbolin-6-carbonsäurechlorid und

3-Ethoxycarbonyl-β-carbolin-6-carbonsäurechlorid.

C) 970 mg 6-Benzyloxycarbonyl-4-ethyl-β-carbolin-3-carbonsäureethylester werden in 50 ml Methanol mit 3 ml 1n Salzsäure und 1,50 g 10%iger Palladium/Kohle bei Raumtemperatur und Normaldruck 1 Stunde hydriert. Nach Abfiltrieren des Katalysators wird eingedampft und man erhält 593 mg 3-Ethoxycarbonyl-4-ethyl-β-carbolin-6-carbonsäure als Hydrochlorid.

In analoger Weise werden als Hydrochloride hergestellt:

3-Ethoxycarbonyl-4-methyl-β-carbolin-6-carbonsäure vom Schmelzpunkt 328—330°C (Ethanol);

3-Ethoxycarbonyl-4-methoxymethyl-β-carbolin-6-carbonsäure;

3-Ethoxycarbonyl-β-carbolin-6-carbonsäure vom Schmelzpunkt 313—314°C (Zersetzung; Ethanol/Essigester) und

3-Ethoxycarbonyl-β-carbolin-5-carbonsäure.

Beispiel 1

1,97 g 6-Jod-4-ethyl-β-carbolin-3-carbonsäureethylester werden unter Kohlenmonoxidatmosphäre in

30 ml Benzylalkohol zusammen mit 1,34 ml Tributylamin auf 100°C erhitzt. Dann werden 55 mg Palladium(II)-acetat zugesetzt, gut mit Kohlenmonoxid gespült und 2 Stunden bei 100°C gerührt. Nach Abdestillieren des Benzylalkohols wird der Rückstand in 300 ml Methylenchlorid aufgenommen, nacheinander mit 80 ml 1-n Salzsäure, 100 ml halbgesättigter Natriumcarbonatlösung sowie 100 ml gesättigter Kochsalzlösung gewaschen, getrocknet, filtriert und eingeengt. Nach Umkristallisation aus Ethanol/Petrolether erhält man 980 mg 6-Benzyloxycarbonyl-4-ethyl-β-carbolin-3-carbonsäureethylester vom Schmelzpunkt 212—215°C.

In analoger Weise werden hergestellt:

6-Benzyloxycarbonyl-4-methyl-β-carbolin-3-carbonsäureethylester;

6-Benzyloxycarbonyl-4-methoxymethyl-β-carbolin-3-carbonsäureethylester vom Schmelzpunkt 183—185°C (Essigester/Hexan);

6-Benzyloxycarbonyl-β-carbolin-3-carbonsäureethylester vom Schmelzpunkt 265—272°C (Dimethylformamid);

6-Butoxycarbonyl-4-methoxymethyl-β-carbolin-3-carbonsäureethylester vom Schmelzpunkt 150—152°C (Ethanol/Hexan) und

3-(5'-[3'-Ethyl-1'.2'.4'-oxadiazol]-yl-β-carbolin-6-carbonsäureethylester vom Schmelzpunkt >330°C (Zersetzung).

Beispiel 2

In eine Suspension von 322 mg 3-Ethoxycarbonyl-4-ethyl-β-carbolin-6-carbonsäurechlorid (als Hydrochlorid) in 10 ml Tetrahydrofuran wird unter Eiskuhlung 10 Minuten Dimethylamin eingeleitet. Danach läßt man auf Raumtemperatur erwärmen und rührt noch 1 Stunde bei Raumtemperatur. Nach Eindampfen wird in Essigester/gesättigter Natriumhydrogencarbonatlösung verteilt und die organische Phase mit 25 ml gesättigter Kochsalzlösung gewaschen, getrocknet, filtriert und eingeengt. Nach Umkristallisation aus Ethanol/Hexan erhält man 175 mg 6-N,N-Dimethylcarbamoyl-4-ethyl-β-carbolin-3-carbonsäureethylester vom Schmelzpunkt 169—170°C.

In analoger Weise werden hergestellt:

6-N.N-Dimethylcarbamoyl-4-methyl-β-carbolin-3-carbonsäureethylester vom Schmelzpunkt 224—225°C (Ethanol/Hexan);

6-N.N-Dimethylcarbamoyl-4-methoxymethyl-β-carbolin-3-carbonsäureethylester vom Schmelzpunkt 131—133°C (Essigester/Diisopropylether) und

6-N.N-Dimethylcarbamoyl-β-carbolin-3-carbonsäureethylester vom Schmelzpunkt 185—196°C.

Beispiel 3

Zu einer Suspension von 372 mg 3-Ethoxycarbonyl-4-ethyl-β-carbolin-6-carbonsäurechlorid (Hydrochlorid) in 10 ml Tetrahydrofuran werden unter Eiskühlung 0,21 ml Diallylamin gegeben. Nach 2 Stunden Rühren wird eingedampft, in Essigester/gesättigter Natriumcarbonatlösung verteilt, die organische Phase getrocknet, filtriert und eingeengt. Nach Chromatographie über Kieselgel mit Methylenchlorid/Ethanol = 10:1 als Elutionsmittel erhält man 266 mg 6-N.N-Diallylcarbamoyl-4-ethyl-β-carbolin-3-carbonsäureethylester als Ol.

In analoger Weise werden hergestellt:

6-N.N-Diethylcarbamoyl-4-methyl-β-carbolin-3-carbonsäureethylester vom Schmelzpunkt 218—220°C (Ethanol/Hexan);

6-N.N-Diallylcarbamoyl-4-methyl-β-carbolin-3-carbonsäureethylester (Öl);

6-Diallylcarbamoyl-4-methoxymethyl-β-carbolin-3-carbonsäureethylester;

6-Diallylcarbamoyl-β-carbolin-3-carbonsäureethylester vom Schmelzpunkt 189—191°C (Cyclohexan/Essigester); und

6-Piperidinocarbonyl-β-carbolin-3-carbonsäureethylester vom Schmelzpunkt 237—240°C.

Beispiel 4

Zu einer Suspension von 320 mg 3-Ethoxycarbonyl-β-carbolin-6-carbonsäurechlorid (Hydrochlorid) in 10 ml Tetrahydrofuran und 8 ml Methylenchlorid werden 83,5 mg Methylammoniumhydrochlorid und 0,6 ml Triethylamin gegeben. Nach Stehen bei Raumtemperatur über Nacht wird Wasser und Eisessig zugegeben, weitgehend eingedampft und in Essigester/gesättigter Natriumhydrogencarbonatlösung verteilt. Die organische Phase wird getrocknet, eingeengt und der Rückstand über Kieselgel mit Methylenchlorid/Ethanol = 10:1 chromatographiert. Nach Umkristallisation der entsprechenden Fraktionen aus Ethanol/Hexan erhält man 25 mg 6-N-Methylcarbamoyl-4-methyl-β-carbolin-3-carbonsäureethylester vom Schmelzpunkt 224—225°C.

Beispiel 5

700 mg 6-Jod-4-methyl-β-carbolin-3-carbonsäureethylester werden in 18 ml Piperidin mit 0,6 ml Tributylamin versetzt und unter Kohlenmonoxidatmosphäre auf 60°C erwärmt. Bei dieser Temperatur fügt man 18 mg Bis[tri-(o-tolyl)-phosphin]palladium(II)-dichlorid zu, spült gut mit Kohlenmonoxid und erhitzt dann den Ansatz 2,5 Stunden auf 80°C. Nach Verdünnen mit Methylenchlorid wird filtriert und eingedampft. Den Rückstand verteilt man in Methylenchlorid und 1n-Salzsäure, wäscht die organische

Phase zweimal mit 1n-Salzsäure, mit verdünntem Ammoniak, mit Wasser, trocknet, filtriert und engt ein. Nach Umkristallisation aus Essigester erhält man 400 mg 6-Piperidinocarbonyl-4-methyl-β-carbolin-3-carbonsäureethylester vom Schmelzpunkt 212—218°C.

Beispiel 6

202 mg 3-Ethoxycarbonyl-4-methoxymethyl-β-carbolin-3-carbonsäure werden in 15 ml Ethanol und 5 ml Wasser mit 206 mg Cäsiumcarbonat in 1,5 ml Wasser versetzt und bis zur klaren Lösung gerührt. Man dampft dann zur Trockne ein, nimmt in 10 ml Dimethylformamid auf, setzt 0,14 ml Methyljodid zu und rührt 3 Stunden bei Raumtemperatur. Nach Eindampfen wird in Methylenchlorid/gesättigter Kochsalzlösung verteilt. Die organische Phase wird getrocknet, filtriert, eingeengt und über Kieselgel mit Methylenchlorid/Ethanol = 6:1 als Elutionsmittel chromatographiert. Nach Umkristallisation der entsprechenden Fraktionen aus Ethanol/Hexan erhält man 92 mg 6-Methoxycarbonyl-4-methoxymethyl-β-carbolin-3-carbonsäureethylester.

In analoger Weise wird:

6-Ethoxycarbonyl-4-methoxymethyl-β-carbolin-3-carbonsäureethylester vom Schmelzpunkt 193°C hergestellt.

Beispiel 7

a.) 345 mg 6-N.N-Dimethylcarbamoyl-β-carbolin-3-carbonsäureethylester werden in 10 ml Ethanol mit 2 ml 1n wässriger Kalilauge 1/2 Stunde am Rückfluß gekocht. Nach dem Abkühlen wird 0,45 ml Eisessig zugetropft und der Ansatz mit wenig Wasser verdünnt. Nach Absaugen und Wachen mit Ethanol und Wasser erhält man 283 mg 6-N.N-Dimethylcarbamoyl-β-carbolin-3-carbonsäure vom Schmelzpunkt 295—297°C (Zersetzung).

In analoger Weise werden hergestellt:

6-N.N-Dimethylcarbamoyl-4-methyl-β-carbolin-3-carbonsäure vom Schmelzpunkt 272°C;

6-Piperidinocarbonyl-4-methyl-β-carbolin-3-carbonsäure vom Schmelzpunkt 291°C (Zersetzung) und

6-Piperidinocarbonyl-β-carbolin-3-carbonsäure vom Schmelzpunkt 271—275°C (Zersetzung).

b.) Eine Mischung von 0,5 g Thionylchlorid in 10 ml absolutem Tetrahydrofuran wird tropfenweise unter Rühren zu einer Lösung von 1,35 g Imidazol in 25 ml Tetrahydrofuran gegeben. Nach 15 Minuten Rühren filtriert man und fügt zu dem Filtrat 0,8 g 4-Methyl-6-N.N-dimethylcarbamoyl-β-carbolin-3-carbonsäure hinzu. Nach 18 Stunden Rühren wird die entstandene Suspension mit 1,25 g Propionamidoxim versetzt und 1 Stunde bei Raumtemperatur gerührt. Nach Stehen über Nacht wird eingedampft, in 30 ml Toluol aufgenommen und 3 Stunden am Rückfluß gekocht. Nach Eindampfen, Verteilen in Methylenchlorid/Wasser, Trocknen, Filtrieren der organischen Phase und Eindampfen der organischen Phase sowie Umkristallisation erhält man 300 mg 4-Methyl-3-(5'-[3'-ethyl-1'.2'.4'-oxadiazol]-yl)-β-carbolin-3-carbonsäuredimethylamid vom Schmelzpunkt 268—272°C.

In analoger Weise erhält man aus β-Carbolin-3-carbonsäure-6-carbonsäureethylester den 3-(5'-[3'-Ethyl-1'.2'.4'-oxadiazol]-yl)-β-carbolin-3-carbonsäureethylester vom Schmelzpunkt >330°C (Zersetzung).

In analoger Weise werden des weiteren hergestellt:

3-(5'-[3'-Ethyl-1'.2'.4'-oxadiazol]-yl)-β-carbolin-3-carbonsäure-N.N-dimethylamid;

3-(5'-[3'-Ethyl-1'.2'.4'-oxadiazol]-yl)-β-carbolin-3-carbonsäurepiperidid vom Schmelzpunkt 291—295°C; und

3-(5'-[3'-Ethyl-1'.2'.4'-oxadiazol]-yl)-4-methyl-β-carbolin-3-carbonsäurepiperidid vom Schmelzpunkt 243—245°C.

Beispiel 8

320 mg 5-Brom-β-carbolin-3-carbonsäureethylester werden in 6 ml Benzylalkohol mit 0,27 ml Tributylamin unter Kohlenmonoxidatmosphäre auf 110°C erhitzt. Dann gibt man 76 mg Palladium-bis(tri-o-tolylphosphin)-dichlorid zu und erwärmt unter Kohlenmonoxid 4 Stunden lang. Anschließend gibt man nochmals 38 mg Katalysator zu und erwärmt 1 Stunde auf 110°C unter Kohlenmonoxid. Nach Eindampfen zur Trockne wird in Dimethylformamid aufgenommen und vom Katalysator abfiltriert. Nach Eindampfen wird der Rückstand über Kieselgel mit Chloroform/Methanol = 10:1,5 als Elutionsmittel chromatographiert. Nach Kristallisation aus Essigester/Diisopropylether erhält man 94 mg 5-Benzyloxycarbonyl-β-carbolin-3-carbonsäureethylester vom Schmelzpunkt 150—155°C.

Beispiel 9

Analog Beispiel 4 wird aus der 3-Ethoxycarbonyl-β-carbolin-5-carbonsäure über die Zwischenstufe des Säurechlorid (siehe Herstellung des Ausgangsmaterials unter Beispiel B) der 5-Piperidinocarbonyl-β-carbolin-3-carbonsäureethylester als ölige Substanz erhalten.

**Patentansprüche**

1. Verfahren zur Herstellung von substituierten β-Carbolinderivaten der allgemeinen Formel I

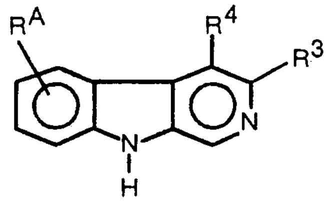

(I),

worin

$R^3$ einen Oxadiazolylrest der Formel

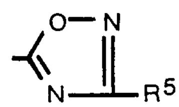

wobei $R^5$ niederes Alkyl mit bis zu 5 C-Atomen bedeutet, oder die Gruppierung —$COOR^6$, wobei $R^6$ niederes Alkyl, mit bis zu 7 C-Atomen bedeutet,

$R^4$ Wasserstoff, niederes Alkyl mit bis zu 3 C-Atomen oder $CH_2OR^9$, wobei $R^9$ niederes Alkyl mit bis zu 3 C-Atomen darstellt, und

$R^A$ die Gruppierung —$COOR^{10}$, wobei $R^{10}$ einen niederen Alkylrest mit bis zu 5 C-Atomen oder die Benzylgruppe darstellt

oder die Gruppierung

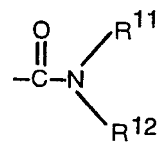

wobei $R^{11}$ und $R^{12}$ gleich oder verschieden sind und Wasserstoff oder niederes Alkyl oder Alkenyl oder gemeinsam einen stickstoffhalgigen 6-gliedrigen Ring darstellen, wobei $R^4$ nicht Wasserstoff oder niederes Alkyl bedeutet, falls $R^A$ und $R^3$ COO-nieder-Alkyl sind, dadurch gekennzeichnet, daß man in an sich bekannter Weise ein substituiertes β-Carbolinderivat der allgemeinen Formel II

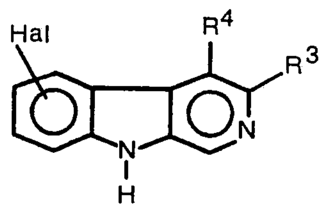

(II),

worin $R^3$ und $R^4$ die oben angegebene Bedeutung haben und Hal Brom oder Jod bedeutet, in Gegenwart einer organischen Base mit Kohlenmonoxid und einem organischen Alkohol der Formel $R^{10}OH$, oder einem stickstoffhaltigen 6-gliedrigem Ring umsetst, wobei $R^{10}$ die obige Bedeutung hat,

oder ein substituiertes β-Carbolinderivat der Formel III

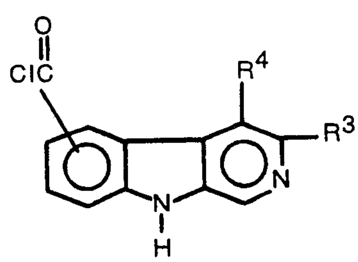

(III),

wobei $R^3$ und $R^4$ die oben angegebene Bedeutung haben, mit einem primären oder sekundären Amin der Formel $HNR^{11}R^{12}$, worin $R^{11}$ und $R^{12}$ die oben angegebene Bedeutung haben, umsetzt

oder eine substituierte β-Carbolin-3-carbonsäure der allgemeinen Formel IV

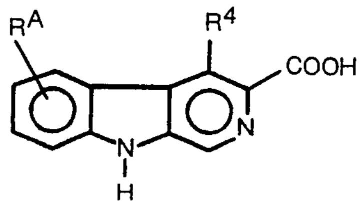

(IV),

worin $R^A$ und $R^4$ die oben angegebene Bedeutung haben, mit einem Amidoxim der allgemeinen Formel $R^5$—C(=NOH)$NH_2$, worin $R^5$ die oben angegebene Bedeutung hat, zum 5-Oxadiazolylderivat umsetzt und gegebenenfalls einen Ester eines aliphatischen Alkohols alkalisch hydrolysiert und gegebenenfalls die freie Säure mit einem Alkohol in gegenwart von Cäsiumcarbonat wieder verestert.

2. β-Carbolinderivate der allgemeinen Formel I

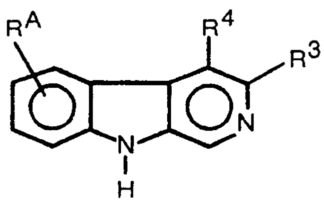

(I),

worin

$R^3$ einen Oxadiazolylrest der Formel

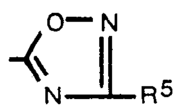

wobei $R^5$ niederes Alkyl mit bis zu 5 C-Atomen bedeutet, oder die Gruppierung —$COOR^6$, wobei $R^6$ niederes Alkyl, mit bis zu 7 C-Atomen bedeutet,

$R^4$ Wasserstoff, niederes Alkyl mit bis zu 3 C-Atomen oder $CH_2OR^9$, wobei $R^9$ niederes Alkyl mit bis zu 3 C-Atomen darstellt, und

$R^A$ die Gruppierung —$COOR^{10}$, wobei $R^{10}$ einen niederen Alkylrest mit bis zu 5 C-Atomen oder die Benzylgruppe darstellt

oder die Gruppierung

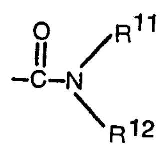

wobei $R^{11}$ und $R^{12}$ gleich oder verschieden sind und Wasserstoff oder niederes Alkyl oder Alkenyl darstellen oder gemeinsam einen stckstoffhaltgigen 6-gliedrigen Ring darstellen, wobei $R^4$ nicht Wasserstoff oder niederes Alkyl bedeutet, falls $R^A$ und $R^3$ COO-nieder-Alkyl sind.

3. β-Carbolin-3.6-dicarbonsäurederivate der Formel V

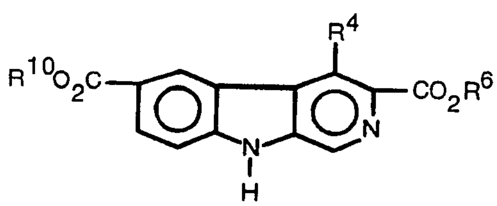

(V),

worin $R^4$, $R^6$ und $R^{10}$ die oben angegebene Bedeutung haben.

4. 6-Aminocarbonyl-β-carbolin-3-carbonsäurederivate der Formel VI

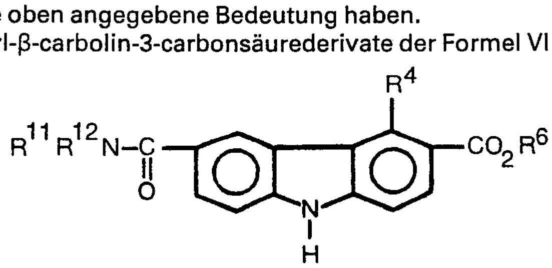

(VI),

worin $R^4$, $R^6$, $R^{11}$ und $R^{12}$ die oben angegebene Bedeutung haben.

5. Arzneimittel auf Basis der Verbindungen gemäß den Ansprüchen 2—4.

**Revendications**

1. Procédé pour préparer des β-carbolines substituées répondant à la formule générale I

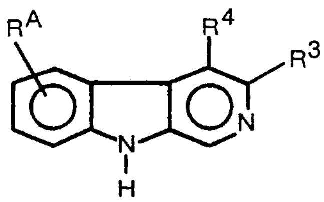

(I),

dans laquelle:

$R^3$ représente un radical oxadiazolyle de formule

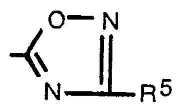

dans lequel $R^5$ désigne un alkyle inférieur renfermant au plus 5 atomes de carbone, ou représente un radical —COOR$^6$ dans lequel $R^6$ désigne un alkyle inférieur contenant au plus 7 atomes de carbone,

$R^4$ représente l'hydrogène, un alkyle inférieur contenant au plus 3 atomes de carbone ou un radical —$CH_2OR^9$ dans lequel $R^9$ désigne un alkyle inférieur contenant au plus 3 atomes de carbone, et

$R^A$ représente un radical —COOR$^{10}$ (dans lequel $R^{10}$ désigne un radical alkyle inférieur contenant au plus 5 atomes de carbone ou un radical benzyle) ou un radical

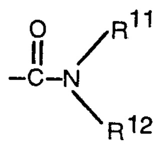

(dans lequel $R^{11}$ et $R^{12}$ représentent chacun, indépendamment l'un de l'autre, l'hydrogène ou un radical alkyle ou alcényle inférieur, ou encore forment ensemble un cycle hexagonal contenant de l'azote),

avec la condition que $R^4$ ne représente ni l'hydrogène ni un alkyle inférieur dans le cas où $R^A$ et $R^3$ représentent un alcoxycarbonyle inférieur,

procédé caractérisé en ce que, en opérant selon des méthodes connues:

on fait réagir une β-carboline substituée de formule générale II:

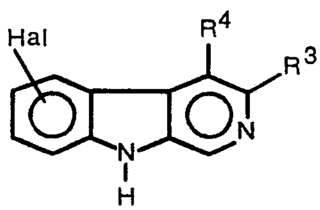

(II),

dans laquelle $R^3$ et $R^4$ ont les significations qui leur ont été données ci-dessus et Hal représente le brome ou l'iode, en présence d'une base organique, avec le monoxyde de carbone et un alcool organique de formule $R^{10}OH$ (dans lequel $R^{10}$ a la signification qui lui a été donnée ci-dessus) ou un composé à cycle hexagonal contenant de l'azote, ou

on fait rèagir une β-carboline substituée de formule générale III:

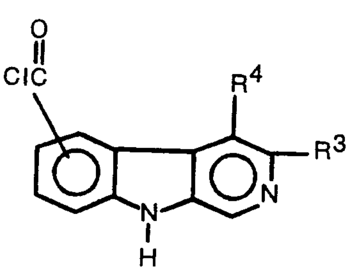

(III),

dans laquelle $R^3$ et $R^4$ ont les significations qui leur ont été données ci-dessus, avec une amine primaire ou secondaire de formule $NHR^{11}R^{12}$ dans laquelle $R^{11}$ et $R^{12}$ ont les significations qui leur ont été données ci-dessus, ou

on fait réagir un acide β-carbolinecarboxylique-3 substitué répondant à la formule générale IV:

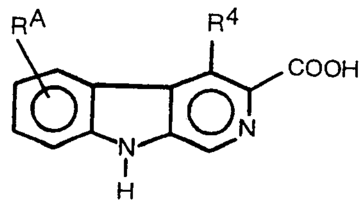

(IV),

dans laquelle $R^A$ et $R^4$ ont les significations qui leur ont été données plus haut, avec une amide-oxime de formule générale $R^5—C(=NOH)NH_2$ dans laquelle $R^5$ a la signification qui lui a été donnée ci-dessus, réaction qui conduit à un composé contenant un radical oxadiazolyle-5,

et éventuellement on hydrolyse en milieu alcalin un ester d'un alcool aliphatique, et éventuellement on estérifie à nouveau l'acide libre avec un alcool en présence de carbonate de césium.

2. β-Carbolines substituées de formule générale I:

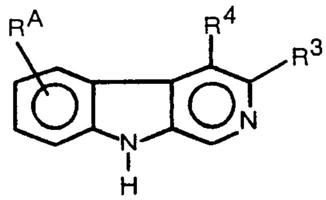

(I),

dans laquelle:

$R^3$ représente un radical oxadiazolyle de formule

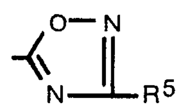

(dans lequel $R^5$ désigne un alkyle inférieur renfermant au plus 5 atomes de carbone) ou représente un radical $—COOR^6$ dans lequel $R^6$ désigne un alkyle inférieur contenant au plus 7 atomes de carbone,

$R^4$ représente l'hydrogène, un alkyle inférieur contenant au plus 3 atomes de carbone ou un radical $—CH_2OR^9$ dans lequel $R^9$ désigne un alkyle inférieur contenant au plus 3 atomes de carbone, et

$R^A$ représente un radical $—COOR^{10}$ (dans lequel $R^{10}$ désigne un radical alkyle inférieur contenant au plus 5 atomes de carbone ou un radical benzyle) ou un radical

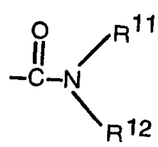

(dans lequel $R^{11}$ et $R^{12}$ représentent chacun, indépendamment l'un de l'autre, l'hydrogène ou un radical alkyle ou alcényle inférieur, ou encore forment ensemble un cycle hexagonal contenant de l'azote),

avec la condition que $R^4$ ne représente ni l'hydrogène ni un alkyle inférieur dans le cas où $R^A$ et $R^3$ représentent un alcoxycarbonyle inférieur.

3. Dérivés de l'acide β-carboline-dicarboxylique-3,6 qui répondent à la formule générale V:

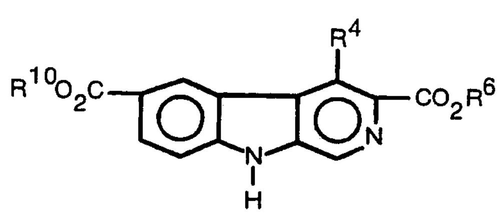

(V),

dans laquelle $R^4$, $R^6$ et $R^{10}$ ont les significations qui leur ont été données plus haut.

4. Dérivés de l'acide carbamoyl-6β-carboline-carboxylique-3 qui répondent à la formule VI:

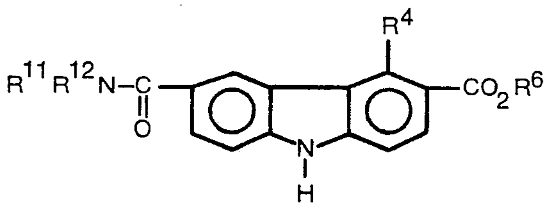

(VI),

dans laquelle $R^4$, $R^6$, $R^{11}$ et $R^{12}$ ont les significations qui leur ont été données ci-dessus.

5. Médicament à base des composés selon l'une quelconque des revendications 2 à 4.

**Claims**

1. Process for the preparaton of substituted β-carboline derivatives of the general formula I

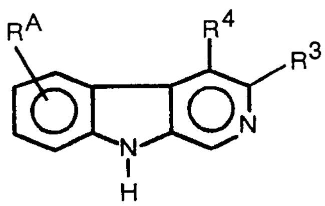

(I),

wherein

$R^3$ is an oxadiazolyl radical of the formula

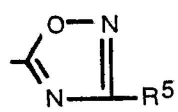

wherein $R^5$ is lower alkyl having up to 5 carbon atoms, or the grouping —$COOR^6$ wherein $R^6$ is lower alkyl having up to 7 carbon atoms,

$R^4$ is hydrogen, lower alkyl having up to 3 carbon atoms or $CH_2OR^9$ wherein $R^9$ is lower alkyl having up to 3 carbon atoms, and

$R^A$ is the grouping —$COOR^{10}$ wherein $R^{10}$ is a lower alkyl radical having up to 5 carbon atoms or a benzyl group, or the grouping

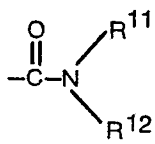

wherein $R^{11}$ and $R^{12}$ are identical or different and are hydrogen or lower alkyl or alkenyl or together are a nitrogen-containing 6-membered ring,

wherein $R^4$ is not hydrogen or lower alkyl if $R^A$ and $R^3$ are COO-lower alkyl,

characterised in that

in a manner known *per se* a substituted β-carboline derivative of the general formula II

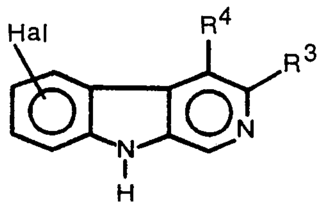

(II),

wherein $R^3$ and $R^4$ have the meanings given above and Hal is bromine or iodine, is reacted in the presence of an organic base with carbon monoxide and an organic alcohol of the formula $R^{10}OH$ or a nitrogen-

containing 6-membered ring, wheein $R^{10}$ has the meaning given above, or

a substituted β-carboline derivative of the formula III

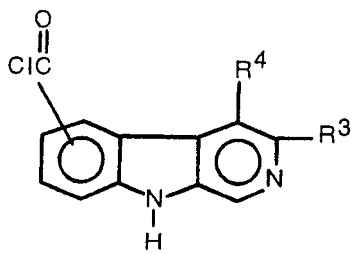

(III),

wherein $R^3$ and $R^4$ have the meanings given above, is reacted with a primary or secondary amine of the formula $HNR^{11}R^{12}$ wherein $R^{11}$ and $R^{12}$ have the meanings given above,

or a substituted β-carboline-3-carboxylic acid of the gneral formula IV

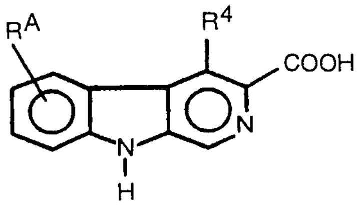

(IV),

wherein $R^A$ and $R^4$ have the meanings given above, is reacted with an amidoxime of the general formula $R^5$—C(=NOH)$NH_2$, wherein $R^5$ has the meaning given above, to form the 5-oxadiazolyl derivative or optionally an ester of an aliphatic alcohol is subjected to alkaline hydrolysis and optionally the free acid is re-esterified with an alcohol in the presence of caesium carbonate.

2. β-Carboline derivative of the general formula I

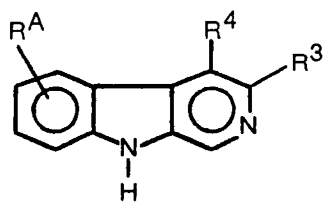

(I),

wherein

$R^3$ is an oxadiazolyl radical of the formula

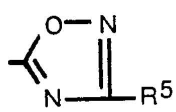

wherein $R^5$ is lower alkyl having up to 5 carbon atoms, or the grouping —$COOR^6$ wherein $R^6$ is lower alkyl having up to 7 carbon atoms,

$R^4$ is hydrogen, lower alkyl having up to 3 carbon atoms or $CH_2OR^9$ wherein $R^9$ is lower alkyl having up to 3 carbon atoms, and

$R^A$ is the grouping —$COOR^{10}$ wherein $R^{10}$ is a lower alkyl radical having up to 5 carbon atoms or a benzyl group, or the grouping

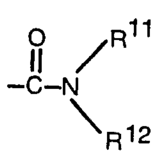

wherein $R^{11}$ and $R^{12}$ are identical or different and are hydrogen or lower alkyl or alkenyl or together are a nitrogen-containing 6-membered ring,

wherein $R^4$ is not hydrogen or lower alkyl if $R^A$ and $R^3$ are COO-lower alkyl.

3. β-Carboline-3,6-dicarboxylic acid derivatives of the formula V

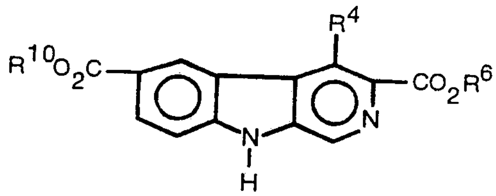

(V),

wherein $R^4$, $R^6$ and $R^{10}$ have the meanings given above.

4. 6-Aminocarbonyl-β-carboline-3-carboxylic acid derivatives of the formula VI

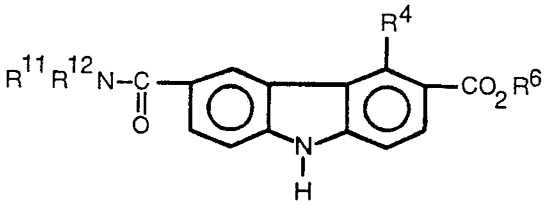

(VI),

wherein $R^4$, $R^6$, $R^{11}$ and $R^{12}$ have the meanings given above.

5. Medicinal agents based on the compounds according to claims 2 to 4.